# EUROPEAN PATENT SPECIFICATION

(11) **EP 3 451 209 B1**
(45) Date of publication and mention of the grant of the patent: **12.08.2020**
(21) Application number: 17188700.3
(22) Date of filing: 31.08.2017
(51) Int. Cl.: G06K 9/00, G06K 9/62, G06N 3/04, G06N 3/08, G06F 21/62

(54) **APPARATUS AND METHOD FOR ANONYMIZING IMAGE CONTENT**
VORRICHTUNG UND VERFAHREN ZUR ANONYMIIERUNG VON BILDINHALT
APPAREIL ET PROCÉDÉ POUR ANONYMIZER UN CONTENU D'IMAGE

(43) Date of publication of application: 06.03.2019
(73) Proprietor: Nokia Technologies Oy, 02610 Espoo (FI)
(72) Inventor: HONKALA, Mikko, 02770 Espoo (FI); KÄRKKÄINEN, Leo, 00100 Helsinki (FI); VETEK, Akos, 00790 Espoo (FI); HUTTUNEN, Janne, 02610 Espoo (FI)
(74) Representative: Nokia EPO representatives

(56) References cited:
- EP-A1- 3 188 058
- US-A1- 2009 169 074
- KHIZAR HAYAT: "Super-Resolution via Deep Learning", ARXIV.ORG, CORNELL UNIVERSITY LIBRARY, 201 OLIN LIBRARY CORNELL UNIVERSITY ITHACA, NY 14853, 28 June 2017 (2017-06-28), XP080773057,

## Description

### Field of invention

The present application relates to apparatus, methods and computer programs for determining and providing anonymised content within images, such as anonymized features within images which maintain other important features, such as medical content.

### Background

Often, there is a requirement to anonymize parts of image content. For example medical images of skin conditions used in teaching may have parts such as faces, body parts anonymized to prevent the image from being associated with a specific person. Furthermore in street view imagery privacy concerns have resulted in parts of the images such as faces, vehicle registration plates being anonymised. Similar privacy issues have meant that airport security images such as generated by back-scattering X-ray machines used in airport security which enable the operator to see under the clothes of the person being scanned are also anonymized. EP3188058 and US2009/169074 describe examples of medical image anonymization.

Anonymization is often achieved by blurring the image. For example Google street view anonymizes images which contain faces or vehicle registration plates by blurring them. Anonymization may also be achieved for example in backscattering X-ray machine images by replacing the 'nude' body image with a cartoon-like representation. However in such current approaches the cartoon-like representations may cause problems. For example it may make the locating of unlawful objects more difficult.

### Summary

In a first aspect there is provided: an apparatus comprising at least one processor, and at least one memory including computer program code, wherein the at least one memory and the computer program code are configured, with the at least one processor, to: train, using a set of training images a generative model network and a discriminator model network to generate a generic image for anonymizing an input image whilst maintaining unprocessed a condition and/or feature; and infer from the input image using the generative model network an output anonymized image.

The set of training images may lack test time identities.

The processor may be configured to train an identifier model network to identify a person from the set of training images and/or a further set of training images, wherein the processor configured to train, using the set of training images the generative model network and the discriminator model network may be further configured to train the generative model network and the discriminator model based on the trained identifier model network.

The processor may be further configured to train a condition model network from the set of training images and/or the further set of training images and/or another set of training images, to identify a condition and/or feature to be maintained unprocessed, wherein the processor configured to train, using the set of training images the generative model network and the discriminator model network may be further configured to train the generative model network and the discriminator model based on the trained condition model network.

The apparatus may be further configured to: check, using the trained identifier model network and the trained condition model network, whether the output anonymized image is successfully anonymized while the condition and/or feature is maintained.

The apparatus may be further configured to configured to infer from the input image using the generative model network a further output anonymized image based on a failed check that the output anonymized image is successfully anonymized while the condition and/or feature is maintained.

The apparatus may be further configured to reject the input image based on a failed check that the output anonymized image is successfully anonymized while the condition and/or feature is maintained.

The condition model network may be a network configured to identify at least one of: a medical condition; a prohibited artefact; and a weapon.

The generative model network and the discriminator model network may be configured as a generative adversarial network.

The generative adversarial network may be at least one of: a Wasserstein generative adversarial network; a boundary equilibrium generative adversarial network; a PixeICNN; and an autoregressive model.

According to a second aspect there is provided a method comprising: training, using a set of training images a generative model network and a discriminator model network to generate a generic image for anonymizing an input image whilst maintaining unprocessed a condition and/or feature; and inferring from the input image using the generative model network an output anonymized image.

The set of training images may lack test time identities.

The method may further comprise training an identifier model network to identify a person from the set of training images and/or a further set of training images, wherein training, using the set of training images the generative model network and the discriminator model network may comprise training the generative model network and the discriminator model based on the trained identifier model network.

The method may further comprise training a condition model network from the set of training images and/or the further set of training images and/or another set of training images, to identify a condition and/or feature to be maintained unprocessed, wherein training, using the set of training images the generative model network and a discriminator model network may comprise training the generative model network and the discriminator model based on the trained condition model network.

The method may further comprise checking, using the trained identifier model network and the trained condition model network, whether the output anonymized image is successfully anonymized while the condition and/or feature is maintained.

The method may further comprise inferring from the input image using the generative model network a further output anonymized image based on a failed check that the output anonymized image is successfully anonymized while the condition and/or feature is maintained.

The method may further comprise rejecting the input image based on a failed check that the output anonymized image is successfully anonymized while the condition and/or feature is maintained.

The condition model network may be a network configured to identify at least one of: a medical condition; a prohibited artefact; and a weapon.

The generative model network and the discriminator model network may be configured as a generative adversarial network.

The generative adversarial network may be at least one of: a Wasserstein generative adversarial network; a boundary equilibrium generative adversarial network; a PixeICNN; and an autoregressive model.

In a further eighth aspect there is provided a computer program comprising program code means adapted to perform the steps of the second aspect when the program is run on a data processing apparatus.

### Brief description of drawings

To assist understanding of the present disclosure and to show how embodiments may be put into effect, reference is made by way of example to the accompanying drawings in which:
Figure 1 schematically shows an apparatus suitable for implementing some embodiments;
Figure 2 schematically shows a zeroth phase system according to some embodiments;
Figure 3 schematically shows a first phase system according to some embodiments;
Figure 4 schematically shows a second phase system according to some embodiments;
Figure 5 schematically shows a flow chart showing the operations of the zeroth, first and second phases according to some embodiments;
Figure 6 shows an example of columns of images being anonymized by blurring and by generic image replacement; and
Figure 7 shows an example of parts of an image being anonymized by generic image replacement.

### Detailed description

The examples disclosed in this application are applicable to apparatus, methods and computer programs for analysing images in order to identify features within an image for anonymizing, wherein anonymizing is configured to generate generic features to replace the identified features. Furthermore the apparatus, methods and computer programs are configured to identify other features and/or conditions shown within the image which are to be preserved accurately.

The concept thus as described in further detail hereafter is one in which deep learning networks may be configured to replace features (or part of the image) that is intended to be anonymized/obfuscated with a machine generated generic (but realistic looking) image part. For instance, a deep learning system may be configured to preserve medically important features, but replace personally identifiable features with generic or random data, but does so in a consistent and reliable manner. A key aspect of the embodiments is the way how multiple machine learning systems are combined in order to consistently anonymize many types of content.

In some embodiments this may be achieved by the use of generative models. Generative models may be trained with missing data and can be configured to provide predictions from inputs that are missing data. Generative adversarial networks in particular, enable machine learning to work with multi-modal output distributions. This for example enables a single input to produce many different correct answers, each of which is acceptable. Some traditional means of training machine learning models, such as minimizing the mean squared error between a desired output and the model's predicted output, are not able to train models that can produce multiple different correct answers.

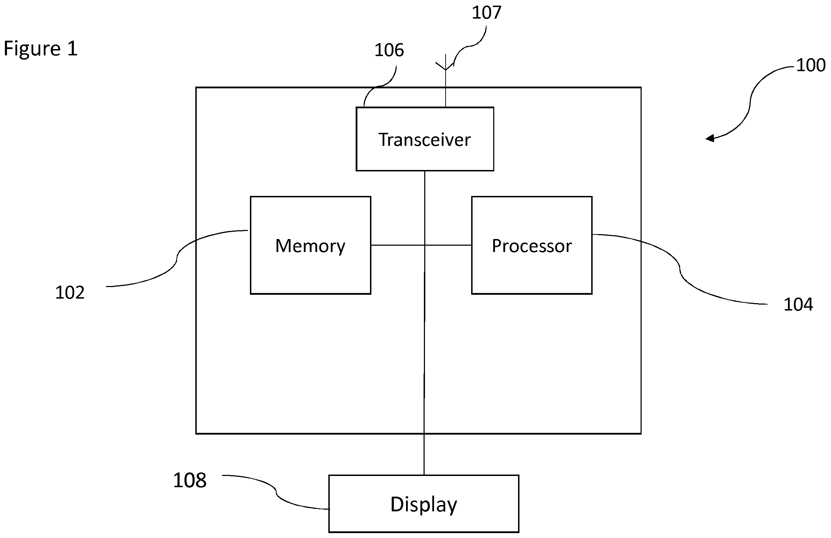

With respect to Figure 1, an example of an apparatus 100 suitable for implementing some embodiments is shown. The apparatus 100 comprises a memory 102 and a processor 104. The apparatus 100 also comprises a transceiver/receiver at 106 for receiving information such as image information from other apparatus or devices. The apparatus 100 transceiver 106 may enable the apparatus 100 to also transmit information e.g. image information to other devices and/or to a remote display. The transceiver 106 may be configured to use any suitable protocol and coding system for the transmitting and/or receiving of information. Furthermore although the transceiver 106 is shown connected to a wireless antenna 107 for communicating wirelessly with further apparatus or devices in some embodiments the transceiver 106 is configured to communicate with the further apparatus or devices at least partially by wired connections.

In some embodiments the apparatus 100 also comprises, or is in communication with, a display 108 which enables the apparatus 100 to provide output in a visual fashion such that it can be interpreted by a viewer. For example the apparatus 100 may cause visual information to be provided on the display 108 which can be viewed by the user such as the medical practitioner, or security officer. In some embodiments the apparatus may furthermore comprise an image source such as a camera or x-ray imaging apparatus suitable for generating the image information to be processed.

In some examples, the apparatus 100 and the processor 104 and memory 102 may be configured to execute code or programs in order to enable the functions and models described hereafter to operate.

Although in the example above the apparatus 100 is shown as a single device and thus operates as a single or centralised apparatus in some embodiments the functionality described hereafter may be enabled by a distributed system of apparatus communicating between each other. For example the functionality may be enabled in a cloud based system wherein apparatus or servers are physically or logically separated from each other.

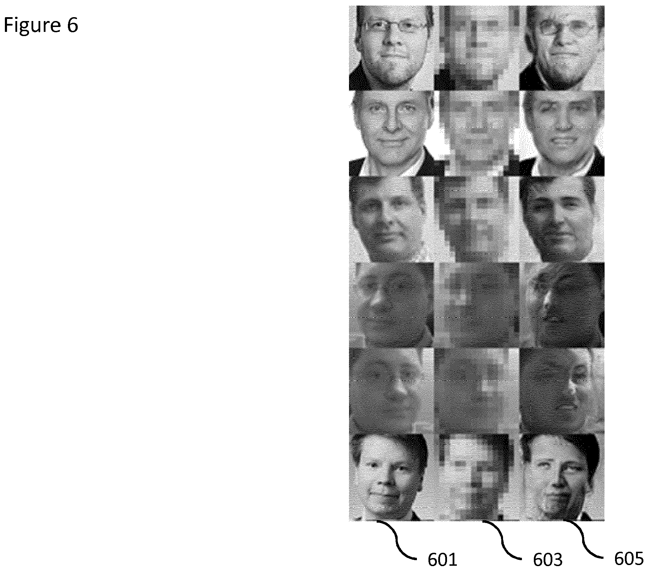

In some embodiments the processor 104 is configured to implement a generative model, using Generative Adversarial Networks (GANs) where the identified parts of an image are processed to replace the identified parts with 'generic' parts. For example as shown in Figure 6 is shown a column 601 of images, each image comprising a face. A second column 603 of images show the effect of a conventional blurring of the faces. As can be seen the effect of the blurring can reduce the information content in elements of the image which are not directly intended to be degraded. For example the pose and the clothing of the image is more difficult to determine from the blurred image column 603 compared to the original image column 601. A third column 605 shows images which have been anonymised by the face features having been replaced by "generic" faces. In some embodiments this may achieved by estimating generative models via an adversarial process, in which two models: a generative model G that captures the data distribution, and a discriminative model D that estimates the probability that a sample came from the training data rather than G are trained at the same time. The training procedure for G may for example be configured to maximize the probability of D making a mistake. This framework may correspond to a minimax two-player game. In the space of arbitrary functions G and D, a unique solution may exist, with G recovering the training data distribution and D equal to 1/2 everywhere. In the case where G and D are defined by multilayer perceptrons, the entire system can be trained with backpropagation. In such implementations there is no need for any Markov chains or unrolled approximate inference networks during either training or generation of samples.

In implementing such anonymising of features the images may be processed in such a manner that the original identity is hidden, while elements such as pose and clothing are kept intact.

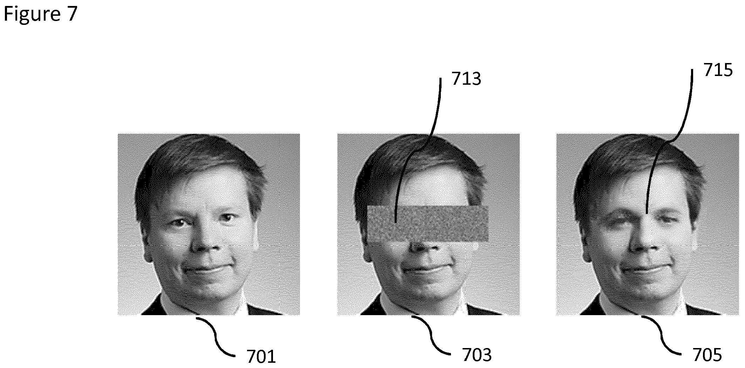

In some embodiments the apparatus may be furthermore be configured to identify parts of an component or only part of the content or certain features of the content for generic image anonymization. For example Figure 7 shows a first image 701 comprising a face, within which a component such as the eyes are identified for anonymization and a further component, such as the mouth, nose or rest of the face are identified as comprising content not to be anonymised. Figure 7 shows a second image 703 with the identified content 713 for anonymization marked. Furthermore Figure 7 shows a third image 705 wherein the identified content is replaced with generic content 715 using a GAN, and here the anonymity is not completely created since large parts of the content is unchanged.

In the following examples the concept is explained with respect to specific example of identifying within image information a person (the component which to be anonymised), and furthermore with respect to the image and the identified person a disease condition (the feature or content not to be anonymised or processed). In such a manner the person's identity may be hidden while enabling medical practitioner to examine the potential disease condition. However it is understood that the same processes as described hereafter may be applied more generally to image data which comprises content to be anonymised by replacement with generic content. Furthermore in some embodiments the content to be anonymised may comprise or may be associated with content, features or conditions within the image which are not to be processed or anonymised in such a manner in order to maintain its specific information content.

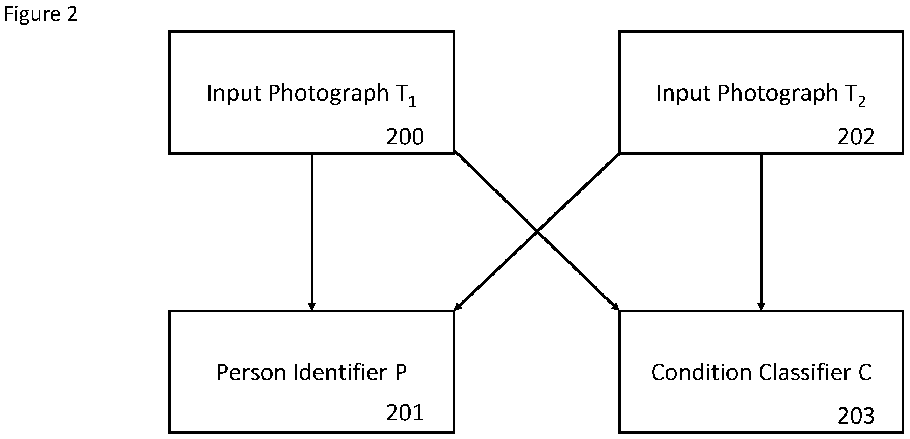

Figures 2 to 5 show example inputs, outputs and the deep learning functions or models which may be implemented by the apparatus as shown in Figure 1 and which implement the example described above of identifying within image information a person (and the content which to be anonymised), and furthermore with respect to the image and the identified person identifying a disease condition (the feature or content not to be anonymised or processed).

In some embodiments therefore there is provided a series of artificial neural networks or learning network models. There may comprise:
a model C which is trained to identify a disease condition;
a model P which is trained to identify persons (using image pairs or multiple images from the same and different persons, .e.g., so called well-known Siamese network); and
a GAN generative model G and discriminator D trained to generate/alter input data in a way that anonymizes the patient by replacing content with generic content.

In some embodiments the models C and P are trained first and they are subsequently used to train models G and D.

In some embodiments the GAN training loss function is extended so that the network is penalized if the disease condition prediction is changed. Furthermore in some embodiments the network is also penalized if the patient is identifiable from the output of the generative model.

Since all networks (even the pre-trained models C and P) are neural networks, the gradient for training may be obtained directly from them. This can be achieved by combining multiple loss functions with some weighted function and derivating it with regards to the model weights.

In some embodiments the networks C and P are kept fixed during the training of the networks G and D. In such embodiments the normal GAN generative loss maintains that the result is indistinguishable from real photographs.

With respect to Figure 2 the inputs and example functions or models implemented in a phase 0 of the example embodiments is shown. Phase 0 occurs prior to the training of the GAN and is primarily concerned with the training of the model C and model P networks.

As such the apparatus, may comprise a first model, model C, which is configured to classify an input image or photograph, for example input photograph T₁ 200 or input photograph T₂202. Model C 203 may be trained to classify the input image or photograph to identify a defined condition orfeature. In some examples the condition may be a specific visual indicator or indication of a defined medical condition, such as a specific pattern of skin colouration.

The apparatus may furthermore comprise a second model, model P, which is trained to identify a person from the input image or photograph, for example input photograph T₁ 200 or input photograph T₂202.

Having trained the models C and P in phase 0 the system may progress to phase 1.

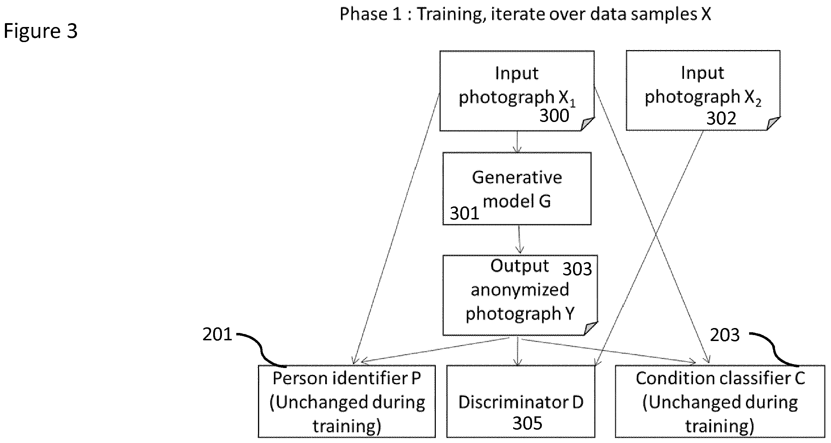

With respect to Figure 3 the example functions or models implemented in a phase 1 of the example embodiments is shown. Phase 1 occurs following the training of the models C and models P and is primarily concerned with the training of the GAN to identify suitable generic image replacement candidates.

As such as shown in Figure 3 example input photograph X₁ 300 (and input photograph X₂ 302) are used as inputs for the phase 1 training. The generative model G 301 may be configured to receive the input photograph X₁ 300 and be configured to output the anonymized photograph Y 303. The output anonymized photograph Y 303 may be passed to the discriminator model D 305. The discriminator model D may be further configured to receive the input photograph X₂302.

Furthermore the output anonymized photograph Y 303 may be passed to the person identifier P 201 (which is unchanged during phase 1 training) and condition classifier model C 203 (which is also unchanged during phase 1 training).

The networks G and D may be trained using the gradients from P 201 and C 203 with regards to the model weights of G and D and in addition to the GAN gradients. This can be accomplished e.g., having a loss function that combines the loss from P and C with the GAN loss using a weighted function and derivating it with regards to the model weights.

Having trained G and D in phase 1 the system may progress to phase 2.

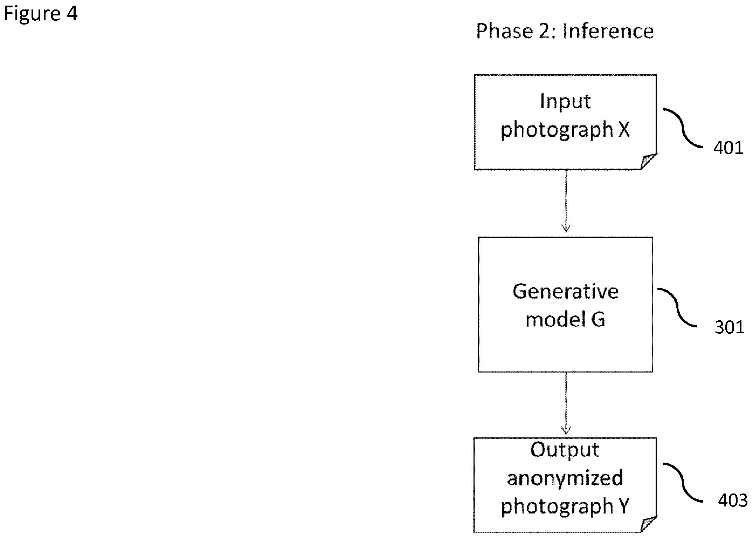

With respect to Figure 4 the example functions or models implemented in a phase 2 of the example embodiments is shown. Phase 2 occurs following the training of the GAN and is primarily the operational mode of the networks configured to identify and replace parts of the image with suitable generic image replacement candidates.

As such as shown in Figure 4 an input photograph X 401 to be processed is passed to the generative model (network) G 301, having been trained in phase 1. The trained generative model G 301 may then process the photograph (image) and generate a suitable anonymized photograph (image) Y 404 wherein at least a portion of the photograph (image) is replaced with an identified 'generic' portion.

In other words in some embodiments phase 2 may be the final usage phase of the model, where only model G is used.

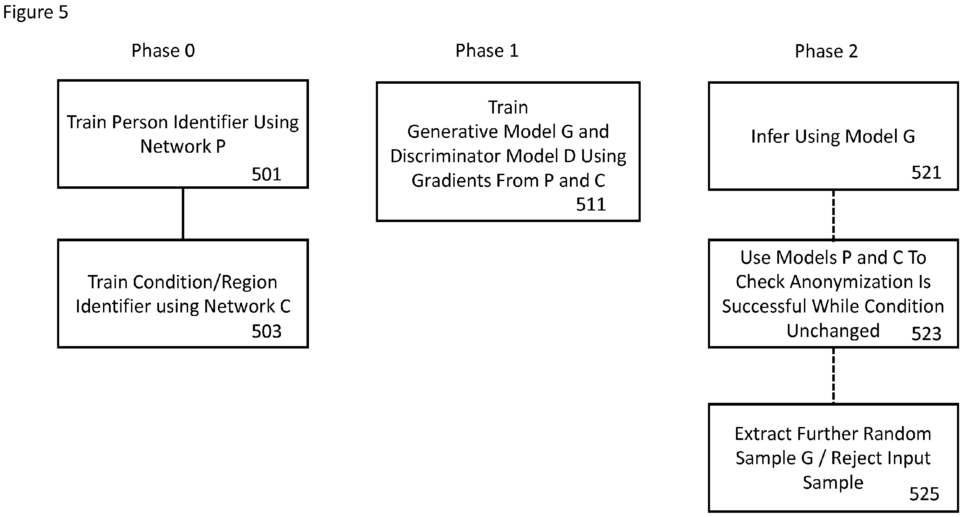

With respect to Figure 5 the operations of the phases shown in Figures 2 to 4 is shown. Furthermore Figure 5 shows a further optional embodiment version of phase 2. In this further optional embodiment models P and C can also be used in the inference phase to make sure that anonymization is successful while the medical condition is unchanged. If this is not the case, then another random sample can be extracted from the generative model or this input sample may be rejected altogether

Thus for example with respect to Figure 5 and phase 0, the apparatus may be configured to train the person identifier using network P for example by using the input photographs T₀ and T₁ or other images.

The operation of training the person identifier using network P is shown in Figure 5 by step 501.

Furthermore the apparatus may be configured to train the condition (or region) identifier using network C for example by using the input photographs T₀ and T₁ or other images.

The operation of training the condition/region identifier is shown in Figure 5 by step 503.

Having trained the networks C and P, then phase 1 may be implemented.

The apparatus may be configured to train the generative model G and discriminator model D using the using the gradients from trained networks P and C with regards to the model weights of G and D, in addition to the GAN gradients.

The operation of training the generative model G and discriminator model D is shown in Figure 5 by step 511.

Having trained the networks G and D, then phase 2 may be implemented.

The apparatus may be configured to infer using trained model G and generate suitable output photograph (images).

The operation of using the trained model G for inferring from the input images and photos is shown in Figure 5 by step 521.

Furthermore in the example shown in Figure 5 in some embodiments the output image or photograph following the operation shown in step 521 is used as an input to models P and C to check for anonymization but maintaining the condition.

The operation of using the models P and C to check anonymization is successful is shown in Figure 5 by step 523.

Should the anonymization be unsuccessful then in some embodiments the apparatus may be configured to extract a further random sample using the trained model G (and furthermore perform an additional anonymization check). In some embodiments where the first or further anonymization operation fails the check then the input sample may be rejected.

The operation of extracting further random samples from model G and/or rejecting the input sample is shown in Figure 5 by step 525.

Although the examples shown above train the four models C,P,D,G in three steps or phases in some embodiments the system may be implemented using fewer models and/or phases.

For example in some embodiments there may be provided a dataset that combines some of the properties. This may be a dataset with medical condition and data of desired output distribution. From such a dataset it may be possible to train the system with fewer models and phases by leaving out the condition classifier model C and training D and G in such a manner that both of them have access to the medical condition of each sample (and thus G learns to retain the condition).

This may be further extended in some embodiments to achieve anonymization by also leaving the person identifier model P out and not allowing the test time identities to exist in the training set. In this example embodiment the level of anonymization cannot be controlled or verified as well as the earlier example embodiments.

As discussed above the system may be used for cases other than medical, such as airport security, the condition classifier C in such embodiments may be replaced by a classifier or segmenter of prohibited artefacts such as weapons. In those cases, the classifier ensures that those artefacts are not altered by the generative model.

Any GAN formulation, such as a Wasserstein generative adversarial network (WGAN), a boundary equilibrium generative adversarial network (BEGAN) may be used as the generative model. In addition to GANs, other methods that allow generative modelling of ambiguous outputs can be used. Some examples of these methods are PixelCNN methods and other autoregressive models.

Implementations using such embodiments thus allow anonymizing medical images and other private data in a way that provides consistent output that is virtually indistinguishable from real data. The output can be stored in databases and even used for further machine learning systems without compromising the identity of the person.

In general, the various embodiments may be implemented in hardware or special purpose circuits, software, logic or any combination thereof. Some aspects of the invention may be implemented in hardware, while other aspects may be implemented in firmware or software which may be executed by a controller, microprocessor or other computing device, although the invention is not limited thereto. While various aspects of the invention may be illustrated and described as block diagrams, flow charts, or using some other pictorial representation, it is well understood that these blocks, apparatus, systems, techniques or methods described herein may be implemented in, as non-limiting examples, hardware, software, firmware, special purpose circuits or logic, general purpose hardware or controller or other computing devices, or some combination thereof.

The embodiments of this invention may be implemented by computer software executable by a data processor of the mobile device, such as in the processor entity, or by hardware, or by a combination of software and hardware. Computer software or program, also called program product, including software routines, applets and/or macros, may be stored in any apparatus-readable data storage medium and they comprise program instructions to perform particular tasks. A computer program product may comprise one or more computer-executable components which, when the program is run, are configured to carry out embodiments. The one or more computer-executable components may be at least one software code or portions of it.

Further in this regard it should be noted that any blocks of the logic flow as in the Figures may represent program steps, or interconnected logic circuits, blocks and functions, or a combination of program steps and logic circuits, blocks and functions. The software may be stored on such physical media as memory chips, or memory blocks implemented within the processor, magnetic media such as hard disk or floppy disks, and optical media such as for example DVD and the data variants thereof, CD. The physical media is a non-transitory media.

The memory may be of any type suitable to the local technical environment and may be implemented using any suitable data storage technology, such as semiconductor based memory devices, magnetic memory devices and systems, optical memory devices and systems, fixed memory and removable memory. The data processors may be of any type suitable to the local technical environment, and may comprise one or more of general purpose computers, special purpose computers, microprocessors, digital signal processors (DSPs), application specific integrated circuits (ASIC), FPGA, gate level circuits and processors based on multi core processor architecture, as non-limiting examples.

Embodiments of the inventions may be practiced in various components such as integrated circuit modules. The design of integrated circuits is by and large a highly automated process. Complex and powerful software tools are available for converting a logic level design into a semiconductor circuit design ready to be etched and formed on a semiconductor substrate.

The foregoing description has provided by way of non-limiting examples a full and informative description of the exemplary embodiment of this invention. However, various modifications and adaptations may become apparent to those skilled in the relevant arts in view of the foregoing description, when read in conjunction with the accompanying drawings and the appended claims. However, all such and similar modifications of the teachings of this invention will still fall within the scope of this invention as defined in the appended claims. Indeed there is a further embodiment comprising a combination of one or more embodiments with any of the other embodiments previously discussed.

## Claims

1. An apparatus comprising at least one processor, and at least one memory including computer program code, **characterized in that** the at least one memory and the computer program code are configured, with the at least one processor, to:
train (501) a first network (201) to identify a person from a set of training images;
train (503) a second network (203), from the set of training images, to identify a condition and/or feature to be maintained unprocessed; and
train (511), using the set of training images or a further set of training images, generative adversarial networks, to generate a generic image for anonymizing an input image whilst maintaining unprocessed the condition and/or feature, wherein the generative adversarial networks comprise a generative model network (310) configured to generate the generic image and a discriminator model network (305) configured for determining whether an image comes from the generative model network or from said set of training images, wherein training the generative model network is configured to maximize the probability of the discriminator model network making a mistake and wherein training the generative model network and the discriminator model network uses a loss function that combines the loss from the trained first network and the trained second network with the loss from the generative adversarial networks, and
based on the training to generate the generic image for anonymizing the input image, infer (521) from the input image, using the generative model network, the generic anonymized image.

2. The apparatus according to claim 1, wherein the apparatus is further configured to:
Check (523), using the first network and the second network, whether the generic anonymized image is successfully anonymized while the condition and/or feature is maintained.

3. The apparatus according to claim 2, wherein the apparatus is further configured to infer from the input image using the generative model network a further generic anonymized image based on a failed check that the generic anonymized image is successfully anonymized while the condition and/or feature is maintained.

4. The apparatus according to claim 3, wherein the apparatus is further configured to reject (525) the input image based on a failed check that the generic anonymized image is successfully anonymized while the condition and/or feature is maintained.

5. The apparatus according to any preceding claim, wherein the second network is a network configured to identify at least one of:
a medical condition;
a prohibited artefact; and
a weapon.

6. The apparatus according to one of the preceding claims, wherein the generative adversarial network is at least one of:
a Wasserstein generative adversarial network;
a boundary equilibrium generative adversarial network;
a PixeICNN; and
an autoregressive model.

7. A computer-implemented method **characterized by** comprising:
training (501) a first network to identify a person from a set of training images;
training (503) a second network, from the set of training images, to identify a condition and/or feature to be maintained unprocessed; and
training (511), using the set of training images or a further set of training images, generative adversarial networks, to generate a generic image for anonymizing an input image whilst maintaining unprocessed the condition and/or feature, wherein the generative adversarial networks comprise a generative model network (310) configured to generate the generic image and a discriminator model network (305) configured for determining whether an image comes from the generative model network or from said set of training images, wherein training the generative model network is configured to maximize the probability of the discriminator model network making a mistake and wherein training the generative model network uses a loss function that combines the loss from the trained first network and the trained second network with the loss from the generative adversarial networks, and
based on the training to generate the generic image for anonymizing the input image, inferring from the input image, using the generative model network, the generic anonymized image.

8. The method according to claim 7, comprising checking, using the first network and the second network, whether the generic anonymized image is successfully anonymized while the condition and/or feature is maintained.

9. The method according to claim 8, further comprising inferring from the input image using the generative model network a further generic anonymized image based on a failed check that the generic anonymized image is successfully anonymized while the condition and/or feature is maintained.

10. The method according to claim 9, further comprising rejecting the input image based on a failed check that the generic anonymized image is successfully anonymized while the condition and/or feature is maintained.

11. The method according to any of claims 7 to 10, wherein the second network is a network configured to identify at least one of:
a medical condition;
a prohibited artefact; and
a weapon.

## Patentansprüche

1. Vorrichtung umfassend mindestens einen Prozessor und mindestens einen Speicher einschließlich Computerprogrammcode, **dadurch gekennzeichnet, dass** der mindestens eine Speicher und der Computerprogrammcode konfiguriert sind, mit dem mindestens einen Prozessor, zum:
Trainieren (501) eines ersten Netzwerks (201), um eine Person aus einer Menge von Trainingsbildern zu identifizieren;
Trainieren (503) eines zweiten Netzwerks (203) aus der Menge von Trainingsbildern, um eine Bedingung und/oder ein Merkmal zu identifizieren, das unverarbeitet gehalten werden soll; und
Trainieren (511), unter Verwendung der Menge von Trainingsbildern oder einer weiteren Menge von Trainingsbildern, von Generative Adversarial Networks, um ein generisches Bild zum Anonymisieren eines eingegebenen Bilds zu generieren, während die Bedingung und/oder das Merkmal unverarbeitet gehalten werden, wobei die Generative Adversarial Networks ein Generative Model Network (310) umfassen, das dazu ausgebildet ist, das generische Bild zu generieren, und ein Discriminator Model Network (305), das dazu ausgebildet ist zu bestimmen, ob ein Bild von dem Generative Model Network oder von der Menge von Trainingsbildern kommt, wobei das Trainieren des Generative Model Networks dazu ausgebildet ist, die Wahrscheinlichkeit zu maximieren, dass das Discriminator Model Network einen Fehler macht, und wobei das Trainieren des Generative Model Networks und des Discriminator Model Networks eine Verlustfunktion verwendet, die den Verlust von dem trainierten ersten Netzwerk und dem trainierten zweiten Netzwerk mit dem Verlust von dem Generative Adversarial Network kombiniert, und
auf Basis des Trainierens, um das generische Bild zum Anonymisieren des eingegebenen Bilds zu generieren, aus dem eingegebenen Bild unter Verwendung des Generative Model Networks auf das generische anonymisierte Bild schließt (521).

2. Vorrichtung nach Anspruch 1, wobei die Vorrichtung weiterhin ausgebildet ist zum:
Prüfen (523), unter Verwendung des ersten Netzwerks und des zweiten Netzwerks, ob das generische anonymisierte Bild erfolgreich anonymisiert ist, während die Bedingung und/oder das Merkmal aufrechterhalten wird.

3. Vorrichtung nach Anspruch 2, wobei die Vorrichtung weiterhin ausgebildet ist zum Schließen von dem eingegebenen Bild unter Verwendung des Generative Model Networks auf ein weiteres generisches anonymisiertes Bild auf Basis einer fehlgeschlagenen Prüfung, dass das generische anonymisierte Bild erfolgreich anonymisiert ist, während die Bedingung und/oder das Merkmal aufrechterhalten wird.

4. Vorrichtung nach Anspruch 3, wobei die Vorrichtung weiterhin dazu ausgebildet ist, das eingegebene Bild auf Basis einer fehlgeschlagenen Prüfung, dass das generische anonymisierte Bild erfolgreich anonymisiert ist, während die Bedingung und/oder das Merkmal aufrechterhalten wird, zurückzuweisen (525).

5. Vorrichtung nach einem vorhergehenden Anspruch, wobei das zweite Netzwerk ein Netzwerk ist, das dazu ausgebildet ist, mindestens einen der Folgenden zu identifizieren:
einen medizinischen Zustand;
ein verbotenes Artefakt; und
eine Waffe.

6. Vorrichtung nach einem der vorhergehenden Ansprüche, wobei das Generative Adversarial Network mindestens eines der Folgenden ist:
ein Wasserstein-Generative Adversarial Network;
ein Boundary Equilibrium Generative Adversarial Network;
ein PixelCNN; und
ein autoregressives Modell.

7. Computerimplementiertes Verfahren, **gekennzeichnet dadurch, dass** es Folgendes umfasst:
Trainieren (501) eines ersten Netzwerks, um eine Person aus einer Menge von Trainingsbildern zu identifizieren;
Trainieren (503) eines zweiten Netzwerks aus der Menge von Trainingsbildern, um eine Bedingung und/oder ein Merkmal zu identifizieren, das unverarbeitet gehalten werden soll; und
Trainieren (511), unter Verwendung der Menge von Trainingsbildern oder einer weiteren Menge von Trainingsbildern, von Generative Adversarial Networks, um ein generisches Bild zum Anonymisieren eines eingegebenen Bilds zu generieren, während die Bedingung und/oder das Merkmal unverarbeitet gehalten werden, wobei die Generative Adversarial Networks ein Generative Model Network (310) umfassen, das dazu ausgebildet ist, das generische Bild zu generieren, und ein Discriminator Model Network (305), das dazu ausgebildet ist zu bestimmen, ob ein Bild von dem Generative Model Network oder von der Menge von Trainingsbildern kommt, wobei das Trainieren des Generative Model Networks dazu ausgebildet ist, die Wahrscheinlichkeit zu maximieren, dass das Discriminator Model Network einen Fehler macht, und wobei das Trainieren des Generative Model Networks eine Verlustfunktion verwendet, die den Verlust von dem trainierten ersten Netzwerk und dem trainierten zweiten Netzwerk mit dem Verlust von dem Generative Adversarial Network kombiniert, und
auf Basis des Trainierens, um das generische Bild zum Anonymisieren des eingegebenen Bilds zu generieren, aus dem eingegebenen Bild unter Verwendung des Generative Model Networks auf das generische anonymisierte Bild schließt.

8. Verfahren nach Anspruch 7, umfassend das Prüfen, unter Verwendung des ersten Netzwerks und des zweiten Netzwerks, ob das generische anonymisierte Bild erfolgreich anonymisiert ist, während die Bedingung und/oder das Merkmal aufrechterhalten wird.

9. Verfahren nach Anspruch 8, weiterhin umfassend das Schließen von dem eingegebenen Bild unter Verwendung des Generative Model Networks auf ein weiteres generisches anonymisiertes Bild auf Basis einer fehlgeschlagenen Prüfung, dass das generische anonymisierte Bild erfolgreich anonymisiert ist, während die Bedingung und/oder das Merkmal aufrechterhalten wird.

10. Verfahren nach Anspruch 9, weiterhin umfassend das Zurückweisen des eingegebenen Bilds auf Basis einer fehlgeschlagenen Prüfung, dass das generische anonymisierte Bild erfolgreich anonymisiert ist, während die Bedingung und/oder das Merkmal aufrechterhalten wird.

11. Verfahren nach einem der Ansprüche 7 bis 10, wobei das zweite Netzwerk ein Netzwerk ist, das dazu ausgebildet ist, mindestens einen der Folgenden zu identifizieren:
einen medizinischen Zustand;
ein verbotenes Artefakt; und
eine Waffe.

## Revendications

1. Appareil comprenant au moins un processeur et au moins une mémoire incluant un code de logiciel, **caractérisé en ce que** l'au moins une mémoire et le code de logiciel sont configurés, à l'aide de l'au moins un processeur, pour :
entraîner (501) un premier réseau (201) à identifier une personne à partir d'un ensemble d'images d'apprentissage ;
entraîner (503) un deuxième réseau (203), à partir de l'ensemble d'images d'apprentissage, à identifier une affection et/ou un élément caractéristique à maintenir non traités ; et
entraîner (511), à l'aide de l'ensemble d'images d'apprentissage ou d'un autre ensemble d'images d'apprentissage, des réseaux contradictoires génératifs à générer une image générique destinée à anonymiser une image d'entrée tout en maintenant non traités l'affection et/ou l'élément caractéristique, les réseaux contradictoires génératifs comprenant un réseau de modèles génératifs (310) configuré pour générer l'image générique et un réseau de modèles discriminants (305) configuré pour déterminer si une image provient du réseau de modèles génératifs ou dudit ensemble d'images d'entraînement, l'entraînement du réseau de modèles génératifs étant configuré pour maximiser la probabilité que le réseau de modèles discriminants fasse une erreur et l'entraînement du réseau de modèles génératifs et du réseau de modèles discriminants utilisant une fonction de perte qui combine la perte du premier réseau entraîné et du deuxième réseau entraîné à la perte des réseaux contradictoires génératifs, et sur la base de l'entraînement à générer l'image générique pour anonymiser l'image d'entrée, déduire (521) de l'image d'entrée, à l'aide du réseau de modèles génératifs, l'image générique anonymisée.

2. Appareil selon la revendication 1, l'appareil étant en outre configuré pour :
vérifier (523), à l'aide du premier réseau et du deuxième réseau, si l'image anonymisée générique est anonymisée avec succès pendant que l'affection et/ou l'élément caractéristique sont maintenus.

3. Appareil selon la revendication 2, l'appareil étant en outre configuré pour déduire de l'image d'entrée, à l'aide du réseau de modèles génératifs, une autre image générique anonymisée sur la base d'un échec de vérification que l'image générique anonymisée est anonymisée avec succès pendant que l'affection et/ou l'élément caractéristique sont maintenus.

4. Appareil selon la revendication 3, l'appareil étant en outre configuré pour rejeter (525) l'image d'entrée sur la base d'un échec de vérification que l'image anonymisée générique est anonymisée avec succès pendant que l'affection et/ou l'élément caractéristique sont maintenus.

5. Appareil selon l'une quelconque des revendications précédentes, le deuxième réseau étant un réseau configuré pour identifier l'un au moins parmi :
une affection médicale ;
un artefact interdit ; et
une arme.

6. Appareil selon l'une des revendications précédentes, le réseau contradictoire génératif étant l'un au moins parmi :
un réseau contradictoire génératif de Wasserstein ;
un réseau contradictoire génératif d'équilibre aux frontières ;
un PixelCNN ; et
un modèle autorégressif.

7. Procédé mis en œuvre par ordinateur, **caractérisé en ce qu'**il comprend :
l'entraînement (501) d'un premier réseau à identifier une personne à partir d'un ensemble d'images d'entraînement ;
l'entraînement (503) d'un deuxième réseau, à partir de l'ensemble d'images d'entraînement, à identifier une affection et/ou un élément caractéristique à maintenir non traités ; et
l'entraînement (511), à l'aide de l'ensemble d'images d'entraînement ou d'un autre ensemble d'images d'entraînement, de réseaux contradictoires génératifs, à générer une image générique pour anonymiser une image d'entrée tout en maintenant non traités l'affection et/ou l'élément caractéristique, les réseaux contradictoires génératifs comprenant un réseau de modèles génératifs (310) configuré pour générer l'image générique et un réseau de modèles discriminants (305) configuré pour déterminer si une image provient du réseau de modèles génératifs ou dudit ensemble d'images d'entraînement, l'entraînement du réseau de modèles génératifs étant configuré pour maximiser la probabilité que le réseau de modèles discriminants fasse une erreur et l'entraînement du réseau de modèles génératifs utilisant une fonction de perte qui combine la perte du premier réseau entraîné et du deuxième réseau entraîné à la perte des réseaux contradictoires génératifs, et
sur la base de l'entraînement à générer l'image générique pour anonymiser l'image d'entrée, la déduction à partir de l'image d'entrée, à l'aide du réseau de modèles génératifs, de l'image générique anonymisée.

8. Procédé selon la revendication 7, comprenant la vérification, à l'aide du premier réseau et du deuxième réseau, que l'image anonymisée générique est anonymisée avec succès pendant que l'affection et/ou l'élément caractéristique sont maintenus.

9. Procédé selon la revendication 8, comprenant en outre la déduction à partir de l'image d'entrée, à l'aide du réseau de modèles génératifs, d'une autre image anonymisée générique, sur la base d'un échec de vérification que l'image générique anonymisée est anonymisée avec succès pendant que l'affection et/ou l'élément caractéristique sont maintenus.

10. Procédé selon la revendication 9, comprenant en outre le rejet de l'image d'entrée sur la base d'un échec de vérification que l'image générique anonymisée est anonymisée avec succès pendant que l'affection et/ou l'élément caractéristique sont maintenus.

11. Procédé selon l'une quelconque des revendications 7 à 10, le deuxième réseau étant un réseau configuré pour identifier l'un au moins parmi :
une affection médicale ;
un artefact interdit ; et
une arme.
